# FASCICULE DE BREVET EUROPEEN

(11) **EP 2 569 628 B1**
(45) Date de publication et mention de la délivrance du brevet: **07.11.2018**
(21) Numéro de dépôt: 11719026.4
(22) Date de dépôt: 12.05.2011
(51) Int. Cl.: G01N 33/50, G06F 19/00

(54) **PROCÉDÉ D'ÉVALUATION TOXICOLOGIQUE, PROCÉDÉ DE CRIBLAGE TOXICOLOGIQUE ET SYSTÈME ASSOCIÉ**
TOXIKOLOGISCHES BEURTEILUNGSVERFAHREN
SYSTEM FOR TOXICOLOGICAL EVALUATION AND TOXICOGENOMICS

(30) Priorité: 12.05.2010 FR 1053732
(43) Date de publication de la demande: 20.03.2013
(73) Titulaire: Université Technologie de Compiègne-UTC, 60203 Compiegne cedex (FR); Centre National de la Recherche Scientifique (CNRS), 75016 Paris (FR); Ecole Normale Supérieure de Lyon, 69007 Lyon (FR); Institut National De L'environnement Industriel Et Des Risques, 60550 Verneuil-en-Halatte (FR)
(72) Inventeur: DUMAS, Marc-Emmanuel, Londres SW8 1JQ (GB); LECLERC, Eric, F-60280 Margny Les Compiegne (FR); SHINTU, Laetitia, F-13005 Marseille (FR); BAUDOIN, Regis, F-93360 Neuilly Plaisance (FR); LEGALLAIS, Cécile, F-60340 Villers Sous Saint Leu (FR); TOULHOAT, Pierre, F-69370 Saint Didier au Mont d'Or (FR); BROCHOT, Céline, F-60340 Villers Sous Saint Leu (FR)
(74) Mandataire: Regimbeau
(86) Numéro de dépôt international: PCT/EP2011/057720
(87) Numéro de publication internationale: WO 2011/151148

(56) Documents cités:
- BORLON CÉLINE ET AL: "The usefulness of toxicogenomics for predicting acute skin irritation on in vitro reconstructed human epidermis.", TOXICOLOGY, vol. 241, no. 3, 30 novembre 2007 (2007-11-30), pages 157-166, XP002614420, ISSN: 0300-483X
- BOLLARD MARY E ET AL: "Metabolic profiling of the effects of D-galactosamine in liver spheroids using (1)H NMR and MAS-NMR spectroscopy.", CHEMICAL RESEARCH IN TOXICOLOGY, vol. 15, no. 11, novembre 2002 (2002-11), pages 1351-1359, XP002614421, ISSN: 0893-228X
- SEAGLE CHRISTOPHER ET AL: "High-throughput nuclear magnetic resonance metabolomic footprinting for tissue engineering.", TISSUE ENGINEERING. PART C, METHODS, vol. 14, no. 2, juin 2008 (2008-06), pages 107-118, XP002614422, ISSN: 1937-3384
- VAN VLIET ET AL: "A novel in vitro metabolomics approach for neurotoxicity testing, proof of principle for methyl mercury chloride and caffeine", NEUROTOXICOLOGY, TOX PRESS, RADFIELD, AR, IN, vol. 29, no. 1, 1 janvier 2008 (2008-01-01), pages 1-12, XP022417840, ISSN: 0161-813X, DOI: DOI:10.1016/J.NEURO.2007.09.007
- Ioro et al: "Statistical Techniques in Metabolic Profiling" In: Balding et al: "Handbook of statistical genetics", 2007, John Wiley & Sons, XP002614423, ISBN: 9780470058305 pages 347-373, 55, para 11.3.1 - pg 357, para 11.3.2
- DAYKIN ET AL: "NMR spectroscopy based metabonomics: current technology and applications", FRONTIERS IN DRUG DESIGN & DISCOVERY, vol. 2, 2006, pages 151-173, XP8130643,
- BRYAN KENNETH ET AL: "MetaFIND: a feature analysis tool for metabolomics data.", BMC BIOINFORMATICS, vol. 9, 470, 2008, pages 1-13, XP002614425, ISSN: 1471-2105
- THYSELL ELIN ET AL: "Reliable profile detection in comparative metabolomics.", OMICS : A JOURNAL OF INTEGRATIVE BIOLOGY SUMMER, vol. 11, no. 2, juillet 2007 (2007-07), pages 209-224, XP002614426, ISSN: 1536-2310
- BAUDOIN ET AL: "Trends in the development of microfluidic cell biochips for in vitro hepatotoxicity", TOXICOLOGY IN VITRO, ELSEVIER SCIENCE, GB, vol. 21, no. 4, 10 avril 2007 (2007-04-10) , pages 535-544, XP022025268, ISSN: 0887-2333, DOI: 10.1016/J.TIV.2006.11.004
- FARKAS DORA ET AL: "In vitro methods to study chemically-induced hepatotoxicity: a literature review.", CURRENT DRUG METABOLISM, vol. 6, no. 2, avril 2005 (2005-04), pages 111-125, XP8094939, ISSN: 1389-2002
- R Baudoin: "Développement et caractérisation d'une puce à cellules pour le criblage d'agents toxiques", HAL, 1 January 2008 (2008-01-01), pages 1-188, XP055467427, DOI: 10.1016/j.comgeo.2016.07.001 Retrieved from the Internet: URL:https://tel.archives-ouvertes.fr/tel-0 0342342/document [retrieved on 2018-04-16]

## Description

### DOMAINE TECHNIQUE GENERAL

La présente invention se rapporte au domaine des bioréacteurs capables de simuler des organes d'organismes vivants tels que des organes de mammifères, en particulier des organes humains.

L'invention concerne l'application de tels bioréacteurs pour le criblage à haut débit de toxiques sur des organes ainsi simulés.

Plus précisément, elle concerne un procédé permettant d'identifier et de quantifier des perturbations métaboliques endogènes liées à la présence de toxiques, grâce à des organes bioartificiels.

### ETAT DE L'ART

Les cultures de cellules représentent à présent un moyen fiable pour réaliser l'évaluation toxicologique de substances chimiques ou biologiques, c'est-à-dire la mesure de leur éventuelle capacité à provoquer des effets néfastes sur une forme de vie.

En concevant des systèmes de culture *in vitro* de cellules d'un tissu ou d'un organe animal ou humain (un tel système, appelé « tissu ou organe bioartificiel », est donc un tissu ou organe synthétique, capable de mimer le comportement biologique du tissu ou de l'organe correspondant dit « naturel » c'est-à-dire pris dans son environnement naturel au sein de l'organisme vivant), on peut prévoir la réaction du tissu ou de l'organe naturel vis-à-vis d'une substance comme un xénobiotique, un cosmétique, un médicament et, plus généralement, n'importe quel principe ou agent potentiellement actif, et ainsi développer des modèles d'étude et d'analyse *in vitro* particulièrement utiles.

Ces modèles sont de plus en plus souvent utilisés dans toutes les phases de la recherche pharmaceutique car ils représentent une alternative avantageuse aux modèles *in vivo,* c'est à dire à l'expérimentation animale, contre laquelle des pressions à la fois économiques et éthiques apparaissent au niveau international. De plus, les coûts associés et la logistique nécessaires à l'expérimentation animale, dans des conditions conformes à la réglementation française et européenne sur le bien-être animal et aux recommandations des services sanitaires compétents, peuvent rendre cette technique prohibitive.

Les méthodes actuelles d'évaluation toxicologique *in vitro* utilisent généralement des boîtes de Pétri. Ces boîtes, contenant un milieu nutritif et placées dans un environnement favorable à la croissance cellulaire, sont ensemencées avec les cellules du tissu ou de l'organe à simuler. Une fois celles-ci développées, elles sont mises en contact avec la substance à tester. L'impact sur les cellules est déterminé via le prélèvement et l'analyse des métabolites, c'est-à-dire les produits de transformation et/ou de dégradation de la substance par les cellules. Cet impact peut être évalué qualitativement, dans ce cas là on parle de « perturbation métabolique », et/ou quantitativement par mesure de marqueurs par exemple, on parle cette fois de « réponse métabolique ». On parle également de « métabolome » pour désigner l'ensemble des métabolites et autres molécules que l'on peut trouver dans un échantillon.

Cependant, ces techniques ne donnent qu'une satisfaction très relative pour déterminer la toxicité d'une molécule particulière et prévoir son impact sur l'organe et, plus généralement, sur l'organisme vivant. En effet, on ne peut se concentrer que sur la recherche de certains métabolites prédéterminés, et donc passer à coté d'autres qui auraient pu être très intéressants. Et une culture cellulaire *in vitro* est inapte à reproduire le comportement d'un tissu ou organe dans son environnement naturel. La cellule cultivée *in vitro* ne se trouve évidemment pas dans le même environnement que lorsqu'elle est au sein du tissu ou de l'organe, *a fortiori* dans l'organisme vivant. Or, les conditions environnementales ont une influence directe sur la ou les réponses biologiques des cellules. Typiquement, une cellule vivante placée dans son environnement naturel intervient dans des voies métaboliques multiples et complexes, qu'il est impossible de reproduire en milieu de culture synthétique. La ou les réponses biologiques de la cellule varieront donc, parfois considérablement, selon qu'elle est cultivée *in vitro* ou qu'elle est dans le tissu ou l'organe et, plus généralement, dans l'organisme vivant. De plus, les cellules sur boîtes de Pétri sont cultivées dans des conditions « statiques » (sans circulation de fluides) et non « dynamiques » (avec circulation de fluides), lesquelles sont pourtant les seules susceptibles de refléter le comportement réel d'un tissu ou organe irrigué.

Pour toutes ces raisons, plusieurs études récentes ont proposé de remplacer en tant que moyen de culture cellulaire les boîtes de Pétri par des bioréacteurs. Ces dispositifs reproduisent un environnement favorable au développement et à l'organisation de cellules, proche de celui d'un tissu ou d'un organe animal ou humain, le plus souvent par des architectures et des matériaux particuliers. Cependant ces systèmes s'avèrent difficilement compatibles avec les techniques d'analyse du métabolome.

Par exemple, un système décrit dans le document « NMR bioreactor development for live in-situ microbial functional analysis », J Magn Reson 192 (1), 159-166 (2008), par Majors, P.D., McLean, J.S., & Scholten, J.C., propose un bioréacteur situé à l'intérieur d'un spectromètre RMN (Résonance Magnétique Nucléaire). La spectrométrie RMN permet d'analyser facilement le milieu de culture du bioréacteur, mais au prix de contraintes très spécifiques. En effet, le bioréacteur se voit interdire les microstructures ainsi que des matériaux autres que le verre, ce qui limite ses possibilités à celles des boîtes de Pétri.

Une autre réalisation, décrite dans le document « Metabolomics for high-resolution monitoring of the cellular physiological state in cell culture engineering », Metab Eng (2009), par Chrysanthopoulos, P.K., Goudar, C.T., & Klapa, M.I, consiste en l'utilisation d'un bioréacteur particulier permettant d'échantillonner et d'analyser les métabolites cellulaires par GC-MS (Gaz Chromatography-Mass Spectrometry). Ce type de spectromètre a prouvé son efficacité dans l'identification de substances, et est notamment utilisé dans les aéroports de monde entier Toutefois, il nécessite que les constituants à identifier soient sous une forme gazeuse. Ainsi, dans le système de Chrysanthopoulos, les métabolomes présents dans le bioréacteur subissent ce que l'on appelle une « dérivatisation », c'est-à-dire la transformation ciblée de certains groupes fonctionnels des molécules pour les rendre plus volatiles. Et cela réduit fortement le nombre de métabolites potentiellement détectables, car elle est restreinte aux métabolites ciblés par la dérivatisation.

Une troisième réalisation, décrite dans le document « High-throughput nuclear magnetic resonance metabolomic footprinting for tissue engineering », Tissue Eng Part C Methods 14 (2008), par Seagle, C., Christie, M.A., Winnike, J.H., McClelland, R.E., Ludlow, J.W., O'Connell, T.M., Gamcsik, M.P., & MacDonald, J.M., consiste en la mesure RMN de métabolites issus de milieux de cultures d'organes bioartificiels.

Cette mesure est suivie d'une analyse statistique. L'idée est de détecter des pics particuliers du spectre RMN obtenu, et donc de « voir » les effets de la substance testée. Cependant, il s'agit d'une analyse univariée rudimentaire du spectre RMN. Seules des effets très forts, très localisés peuvent être détectés, une grande part de l'information est perdue. La détection ciblée reste nécessaire, avec toutes les incertitudes qu'elle comporte, si l'on souhaite évaluer finement une perturbation métabolique

Finalement, aucun de ces travaux ne peut trouver une réelle application en évaluation toxicologique, les performances n'étant pas au rendez-vous.

De plus ceux-ci ne sont pas directement applicables à l'analyse « inverse » qu'est le criblage toxicologique, une méthode permettant d'identifier une substance inconnue à travers ses effets toxicologiques connus. Ce criblage n'est aujourd'hui possible que par comparaison, c'est-à-dire en observant les effets de la substance (*in vivo* ou *in vitro*), et en comparant avec les effets connus de diverses substances jusqu'à trouver la plus proche avec un degré de confiance suffisant.

Pour toutes ces raisons, les techniques *in vitro* n'ont pas encore supplanté les techniques *in vivo,* et restent encore à améliorer.

Le document Borlon et al, 2007. Toxicol, 241, 157-166 décrit un procédé d'évaluation toxicologique à l'aide d'une puce à ADN pour l'observation de la réponse métabolique.

Le document Bollard et al, 2002. Chem Res Roxicol, 15, 1351-1359 décrit un procédé d'évaluation toxicologique utilisant une RMN dite à « magic angle spinning » destinée à analyse des échantillons solides.

Le document van Vliet et al, 2008. Neurotox, 29(1), 1-12 décrit la culture de cellules neuronales en solution.

Les documents Iorio et al, 2007. Handbook of Statistical Genetics, Chapter 11, pp 347-373, Daykin et al, 2006. Front Drug Design & Disc, 2, 151-173, Bryan et al, 2008. BMC Bioinformatics, 9, 470, et Thysell et al, 2007. Omics, 11(2), 209-224, décrivent l'utilisation de la technique de PLS-DA.

Le document Baudoin et al, 2007. Toxicol in Vitro, 535-544, décrit l'observation de la réponse métabolique d'un tissu avec une puce à ADN.

Le document Farkas et al, 2005. Curr Drug Metab, 6(2), 111-125 décrit différentes méthodes d'observation de la réponse métabolique d'un tissu.

Le document Baudoin, 2008. thesis. HAL, 1-188, décrit l'observation de la réponse métabolique d'un tissu.

### PRESENTATION DE L'INVENTION

La présente invention vise à résoudre ces difficultés en proposant un procédé d'évaluation toxicologique sur des tissus ou organes bioartificiels simulant parfaitement des organes réels, et proposant une nouvelle approche d'analyse de la réponse métabolique.

Un but annexe de l'invention est de parvenir à cet objectif tout en proposant un procédé plus rapide et moins coûteux à mettre en oeuvre, plus fiable, offrant des résultats parfaitement reproductibles et bien plus facilement exploitables.

La présente invention se rapporte donc à un procédé d'évaluation toxicologique d'une substance candidate sur au moins un tissu ou organe, caractérisé en ce qu'il comprend les étapes de :
(a) Obtention d'un tissu ou organe bioartificiel par simulation ou modélisation de l'activité métabolique dudit tissu ou organe par plusieurs bioréacteurs montés en série ou en parallèle et dans lesquels sont cultivées à la suite différentes familles de cellules qui ensemble simulent le tissu ou l'organe ;
(b) Exposition dudit tissu ou organe bioartificiel à ladite substance ;
(c) Observation de la réponse métabolique du tissu ou de l'organe bioartificiel et acquisition d'un jeu de données multidimensionnel associé sans émettre d'hypothèses concernant la réponse métabolique du tissu ;
(d) Identification au moyen d'une méthode d'analyse statistique multivariée des composantes du jeu de données multidimensionnel quantitativement corrélées à des variables prédéterminées ;
(e) Génération d'un modèle prédictif à partir des composantes du jeu de données effectivement retenues ;
(f) Test du caractère prédictif dudit modèle par au moins une méthode statistique d'estimation de fiabilité ;
(g) Identification de la réponse métabolique du tissu ou de l'organe bioartificiel sous la forme de biomarqueurs associés aux composantes du jeu de données retenues pour le modèle.

L'évaluation classique par prélèvement des substances toxiques et de leurs résidus (c'est à dire des métabolites xénobiotiques), dite directe, se fait par méthodes enzymatiques ou spectrométrie de masse couplée ou non à une méthode séparative de type chromatographie. Ces méthodes sont dites ciblées, ou « avec a priori » car elles font appel à la détection de substances pour lesquelles un protocole de détection spécifique est mis en oeuvre. En revanche, ces méthodes ne sont pas appropriées pour la détection et l'identification de marqueurs des effets d'une substance inconnue, comme expliqué précédemment.

Afin de répondre à cette problématique, l'invention se propose d'étudier les conséquences indirectes de l'exposition des organes bioartificiels à une substance toxique, en caractérisant la signature métabolique de la réponse à cette substance.

L'invention se focalise sur l'utilisation de méthodes dites « non ciblées » de type spectroscopie par Résonance Magnétique Nucléaire (RMN) ou spectrométrie de masse (SM), avec ou sans couplage chromatographique, afin de détecter le plus grand nombre possible de métabolites endogènes. Afin d'identifier une réponse globale à l'échelle du métabolisme, ces différentes mesures parallèles sont alors analysées par statistiques multidimensionnelles. Ces analyses visent en fait à « expliquer » les paramètres de l'expérience grâce aux milliers de variables mesurées, et d'en déduire un modèle de prédiction. Il est en effet possible d'utiliser des techniques de reconnaissance de formes supervisées telles que l'analyse discriminante, la régression aux moindres carres partiels (ou régression PLS), les réseaux de neurones artificiels, etc.

Selon d'autres caractéristiques avantageuses et non limitatives :
- la méthode d'analyse multivariée utilisée à l'étape (d) est une analyse discriminante par régression aux moindres carrés partiels (PLS-DA) ;
- l'étape (d) est précédée d'une étape (d1) de repérage et d'élimination des points aberrants dans ledit jeu de données multidimensionnel ;
- l'étape (d1) comprend une analyse non supervisée par composantes principales (PCA) ;
- l'une des méthodes statistiques d'estimation de fiabilité utilisée à l'étape (d) est une validation croisée ;
- l'une des méthodes statistiques d'estimation de fiabilité utilisée à l'étape (d) est une validation par l'hypothèse nulle ;
- l'une des méthodes statistiques d'estimation de fiabilité utilisée à l'étape (d) comprend le calcul de l'aire sous une courbe Receiver Operative Characteristic (ROC) ;
- les variables prédéterminées utilisées à l'étape (d) sont choisies parmi les conditions de culture, le type cellulaire du tissu ou de l'organe bioartificiel, le type de ladite substance, et sa dose ;
- l'étape (c) s'effectue par au moins une technique de chimie analytique choisie, parmi les techniques du groupe comprenant la spectroscopie RMN du proton, la spectroscopie RMN du carbone, la spectrométrie de masse, la chromatographie en phase gazeuse, la chromatographie en phase liquide, et les méthodes de détection multiplexées ;
- l'étape (c) s'effectue dans le milieu de culture en sortie de bioréacteur afin d'observer la réponse métabolique extracellulaire ;
- l'étape (c) s'effectue sur les culots cellulaires afin d'observer la réponse métabolique endogène intracellulaire ;
- le procédé comprend en outre une étape (h) d'obtention d'une signature de toxicité spécifique de ladite substance à partir de la liste des biomarqueurs obtenue à l'étape (g) ;
- le procédé comprend en outre une étape (i) dans laquelle on renseigne une banque de marqueurs et/ou de signatures de toxicité à l'aide des informations obtenues lors des étapes précédentes.

La présente invention vise aussi à permettre un criblage toxicologique fiable à très haut débit, selon un deuxième aspect.

La présente invention se rapporte donc en outre à un procédé de criblage toxicologique d'une substance candidate sur au moins un tissu ou organe, caractérisé en ce qu'il comprend les étapes de :
(a) Obtention d'un tissu ou organe bioartificiel par simulation ou modélisation de l'activité métabolique dudit tissu ou organe par plusieurs bioréacteurs montés en série ou en parallèle et dans lesquels sont cultivées à la suite différentes familles de cellules qui ensemble simulent le tissu ou l'organe ;
(b) Exposition dudit tissu ou organe bioartificiel à ladite substance ;
(c) Observation de la réponse métabolique du tissu ou de l'organe bioartificiel et acquisition d'un jeu de données multidimensionnel associé sans émettre d'hypothèses concernant la réponse métabolique du tissu ;
(d) Identification au moyen d'une méthode d'analyse statistique multivariée des composantes du jeu de données multidimensionnel quantitativement corrélées à des variables prédéterminées ;
(e) Génération d'un modèle prédictif à partir des composantes du jeu de données effectivement retenues ;
(f) Test du caractère prédictif dudit modèle par au moins une méthode statistique d'estimation de fiabilité ;
(g) Identification de la réponse métabolique du tissu ou de l'organe bioartificiel sous la forme de biomarqueurs associés aux composantes du jeu de données retenues pour le modèle ;
(h) Obtention d'une signature de toxicité spécifique de ladite substance à partir de la liste des biomarqueurs obtenue à l'étape (g)
(i) Comparaison des biomarqueurs et/ou de la signature de toxicité spécifique avec une banque de marqueurs et/ou signatures de toxicité, de manière à identifier ladite substance.

Selon d'autres caractéristiques avantageuses et non limitatives : - ladite banque de marqueurs et/ou signatures de toxicité a été établie par la mise en oeuvre d'au moins un procédé selon le premier aspect de l'invention.

Ce criblage à haut débit de substances est rendu possible via la constitution d'une base ou banque de données compilant les résultats d'expériences préalables. Cette application ouvre la porte au criblage analytique de substances dont la toxicité n'aurait jamais été démontrée, par comparaison rapide, immédiate et statistique avec des substances de référence, dont on connaît la signature de toxicité, c'est-à-dire une représentation spécifique de la réponse métabolique.

L'invention vise enfin des systèmes, l'un comprenant une pluralité de bioréacteurs, montés en série ou en parallèle et dans lesquels sont cultivées à la suite différentes familles de cellules qui ensemble simulent le tissu ou l'organe, des moyens de traitement de données et un dispositif de détection de biomarqueurs, caractérisé en ce qu'il adapté pour mettre en oeuvre un procédé d'évaluation toxicologique selon le premier aspect de l'invention, et l'autre comprenant en outre des moyens de stockage de données, caractérisé en ce qu'il adapté pour mettre en oeuvre un procédé de criblage toxicologique selon le deuxième aspect de l'invention.

### PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :
- la figure 1 est un exemple de spectre ¹H RMN (RMN du proton) ;
- la figure 2 est un graphique illustrant le résultat d'une première expérience comparant un procédé d'évaluation toxicologique selon l'invention et un procédé similaire qui utiliserait des boîtes de Pétri grâce a une modélisation des spectres ¹H RMN par régression aux moindres carrés partiels ;
- la figure 3 est un graphique illustrant le résultat d'une seconde expérience comparant différents types cellulaires par ¹H RMN et régression PLS ;
- les figures 4ac sont trois graphiques illustrant le résultat d'une troisième expérience comparant une réponse métabolique à différentes doses d'un même xénobiotique par analyse ¹H RMN et régression PLS ;
- les figures 5a-b sont deux exemples de courbes Receiver Operating Characteristic utilisées dans une mode de réalisation du procédé d'évaluation toxicologique selon l'invention.

### DESCRIPTION DETAILLEE

### Evaluation toxicologique

Le procédé d'évaluation toxicologique selon le premier aspect de l'invention commence par une étape d'obtention d'un tissu ou organe artificiel, sur lequel la substance va être testée. Pour cela, des bioréacteurs sont utilisés. Ceux-ci comprennent avantageusement une chambre de culture comportant une paroi supérieure et une paroi inférieure microstructurées favorisant le développement cellulaire, un point d'entrée de fluide et un point de sortie de fluide pour permettre le passage d'un fluide nutritif nécessaire au développement et à la croissance des cellules et à terme l'exposition à la substance dont on souhaite étudier les propriétés toxicologiques. De tels bioréacteurs sont décrits de manière détaillée dans la demande de brevet FR0954288 déposée le 23 juin 2009, à laquelle on fait ici référence. L'invention n'est toutefois pas limitée à ce type de bioréacteur en particulier.

Les microstructures d'un bioréacteur rendent possible le développement de tissus ou d'organes bioartificiels présentant une structure cellulaire élaborée conforme à la réalité. La croissance est accélérée et autonome. Les bioréacteurs se montent facilement en série ou en parallèle. Il est ainsi possible de cultiver à la suite différentes familles de cellules, qui ensemble simulent un organe. Les organes vivants sont en effet des systèmes très complexes qui abritent en général un type de cellules principal, plus de nombreuses autres, dont la présence d'avère essentielle. Par exemple, dans le cas du foie 80 % des cellules sont ce que l'on appelle des hépatocytes. Mais l'on trouve également des cellules endothéliales, des cellules de Küppfer, des cellules de Ito, des lymphocytes hépatocytaires...

Plus simplement, il est aussi possible de viser plutôt une reproduction structurelle de la fonction de l'organe, et par là de le modéliser. On peut citer par exemple le cas du rein.

Par ailleurs, le fonctionnement dynamique du bioréacteur permet, outre une meilleure simulation du tissu ou de l'organe, d'exposer à une substance puis de récupérer facilement la réponse métabolique des cellules cultivées au niveau de la connectique de sortie du bioréacteur. Dans ce cas là, on observe la réponse métabolique dite extracellulaire. Alternativement il peut être utile d'observer la réponse métabolique endogène, c'est-à-dire à l'intérieur même des cellules. Dans ce cas là on récupère la réponse métabolique directement sur les culots cellulaires.

Les tissus ou organes bioartificiels sont donc cultivés jusqu'à maturité, puis exposé à une substance à tester. Après un temps prédéterminé selon le protocole du test, des échantillons sont récupérés, que ce soit en sortie à l'intérieur des cellules.

Les échantillons sont préparés pour observation. Comme expliqué, les méthodes dites « non ciblées » du type RMN ou spectrométrie de masse sont particulièrement préférées. Ces méthodes ainsi que de nombreuses autres, sont largement utilisées en chimie analytique et connues de l'homme du métier. Un spectre RMN d'un échantillon de milieu de culture d'un organe bioartificiel permet par exemple une acquisition en parallèle et sans *a priori* de signaux de plusieurs dizaines, voire centaines de métabolites, c'est-à-dire une acquisition multidimensionnelle. La RMN est une méthode de spectroscopie appliquée à une particule ou à un ensemble de particules. En particulier, les RMN basées sur des atomes caractéristiques des molécules organiques, comme l'hydrogène ou le carbone s'appliquent particulièrement à l'invention.

Par acquisition « sans a priori » d'un jeu de données associé à la réponse métabolique, on comprendra acquisition de toute donnée associée à une quelconque réponse métabolique, et non seulement des données associées à des éléments « attendus » d'une réponse métabolique. Aucune hypothèse concernant les métabolites impliqués dans la réponse biologique ou toxicologique d'intérêt n'est à faire. En d'autres termes, une acquisition sans a priori d'un jeu de données associé à la réponse métabolique est une acquisition qui incorpore y compris les données correspondant à des métabolites inconnus. Une approche sans a priori se différencie fondamentalement de l'approche ciblée ("targeted metabolomics") dans laquelle tout ce qui ne correspond pas aux métabolites ciblés est ignoré.

Comme expliqué précédemment, l'utilisation de RMN ou d'autres méthodes de spectroscopie entre autres permet une acquisition sans a priori, contrairement à l'utilisation par exemple d'une puce à ADN, qui limite à l'acquisition de données associées à l'expression des seuls gènes que comporte la puce, en d'autres termes à une réponse métabolique partielle. Les statistiques multidimensionnelles permettent ensuite d'identifier les signaux métaboliques significativement affectés et dans quelles proportions. En conséquence, l'approche sans a priori présente un fort intérêt pour découvrir des nouveaux marqueurs associés à cette réponse toxicologique. Dans l'approche ciblée, le potentiel en termes de découverte de nouveaux marqueurs est inexistant.

Dans le mode de réalisation préféré décrit, on utilise la spectroscopie ¹H RMN (RMN du proton, qui permet de détecter les atomes d'hydrogène), avec laquelle une série de tests ont été réalisés. Ces tests ont impliqué deux lignées de cellules et trois substances potentiellement toxiques, les deux lignées pouvant être éventuellement en co-culture. Ces cellules sont d'un coté des HEPG2/C3A, simplement nommées C3A, des cellules hépatocytes humaines, et de l'autre coté des MDCK (Madin-Darby Canine Kidney Cells), des cellules de rein du chien. On notera que l'invention n'est nullement limitée à des organes bioartificiels de type foie ou rein, et que l'homme du métier pourra transposer l'invention à tout type d'organe bioartificiel simulable ou modélisable en bioréacteur. En particulier, on peut citer le pancréas, le coeur, les testicules, des parties du cerveau, etc.

Les xénobiotiques auxquels ont été exposées les cellules utilisées dans les tests sont l'ammoniac (NH3), le Diméthysulfoxyde (DMSO) et le N-acétyl-para-aminophénol (APAP), plus connu sont le nom de paracétamol. Ces trois substances sont connues pour avoir des effets nocifs sur le foie à haute dose.

Les échantillons de milieu sont par exemple préparés en utilisant 350 µL de milieu, mélange avec 200 µL de solution saline a 0.9 g/L composée a 90% d'eau (H2O) et de 10% d'eau lourde (D2O) à des fins de calibration. Les 550 µL de solution sont ensuite transférés dans un tube d'analyse RMN.

L'acquisition RMN est faite à une fréquence de 700 MHz en utilisant une sonde proton. Un spectre RMN 1D est enregistré en utilisant une pré-saturation de la résonance de l'eau suivant la séquence suivante : T_{rd}-P₉₀-t₁-P₉₀-tₘ-P₉₀-T_{aq}. Trd représente un délai de 2s durant lequel la résonance de l'eau est sélectivement irradiée, P90 est une impulsion de radiofréquence de 90° et t1 correspond à un délai de 3 µs. La résonance de l'eau est irradiée une deuxième fois pendant le temps de mélange (tₘ=100ms) et un temps d'acquisition de 1.95s. Pour chaque échantillon, 128 interférogrammes sont accumulés en vue d'augmenter le rapport signal/bruit. Ces interférogrammes sont multipliés par une fonction exponentielle correspondant à un élargissement de raie de 0.3Hz. Préalablement à l'obtention d'une transformée de Fourier, la largeur de l'interférogramme est doublée en ajoutant des points nuls en fin d'interférogramme, afin de ne pas dégrader la résolution du spectre. L'échelle dans la dimension horizontale correspond aux déplacements chimiques ¹H. Elle s'étend sur une fenêtre de 10 ppm autour de la résonance de l'eau. L'échelle dans la dimension verticale correspond à l'intensité des résonances qui est proportionnelle au nombre de protons présents pour un groupement chimique de chaque molécule de l'échantillon. Les spectres sont alors phasés et une correction linéaire de ligne de base est effectuée, avant calibration sur le signal de l'anomère beta du glucose à 5.23ppm. La région entre 4.67ppm et 5ppm autour du signal résiduel de l'eau est supprimée pour une meilleure visibilité, et les spectres sont exportés en tant que jeu de données multimidimensionnels.

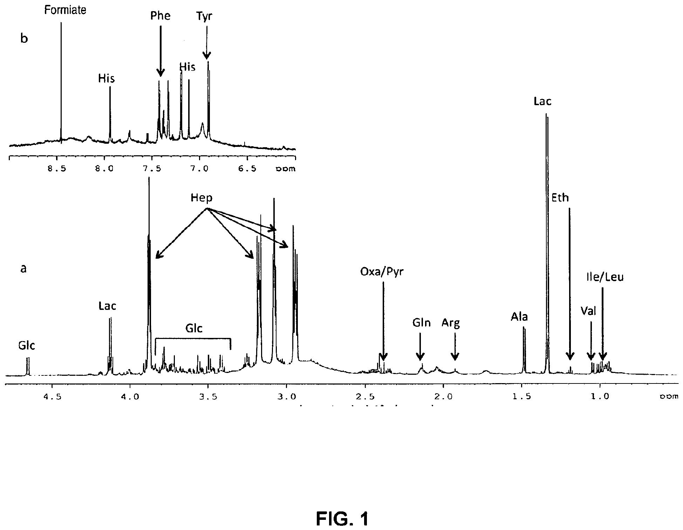

La figure 1 montre un exemple de spectre ¹H RMN d'un échantillon de surnageant de culture de cellules C3A exposées à l'ammoniac (25µl/min, 10 mM NH₃). La région comprenant les résonances aromatiques (entre 6.5 et 10 ppm) a été agrandie. Les pics des métabolites principaux sont annotés à titre d'exemple. Légende: His: histidine, Phe: phénylalanine, Tyr: tyrosine, Glc: glucose, Lac: lactate, Oxa: oxaloacétate, Pyr: pyruvate, Gln: Glycine, Arg: Arginine, Ala: alanine, Eth: éthanol, Val: valine, Leu: leucine, Ile:Isoleucine.

L'analyse statistique consiste tout d'abord en une analyse non supervisée par composantes principales afin de repérer les points aberrants dans le jeu de données multidimensionnel. L'analyse en composante principale permet de compresser les 40,000 RMN redondantes présentes dans le jeu de données dans un nouveau repère orthonormé, chaque axe représentant une composante principale de la variance du jeu de donnée, qui est donc multidimensionnel. Une analyse statistique multivariée, dite supervisée, est alors effectuée afin de discriminer conditions de culture ou les groupes de doses, et identifier les signatures métaboliques spécifiques aux effets toxicologiques. Cette analyse supervisée est principalement effectuée grâce à la régression aux moindres carrés partiels (Partial Least Squares), à travers l'analyse discriminante par régression aux moindres carrés partiels, connue sous les initiales PLS-DA, sans toutefois s'y limiter. Une régression PLS, tout comme la régression linéaire, permet d'identifier les composantes du jeu de données (variables explicatives, X) qui sont quantitativement corrélées à la variable à expliquer (Y), que ce soient des conditions de culture, le type cellulaire de l'organe bioartificiel, le type de traitement administré ou la dose du traitement. Des structures de réseaux de neurones artificiels peuvent également être programmées pour effectuer cette analyse multivariée.

Afin de tester le caractère prédictif des modèles supervisés (modèles PLS dans le cas présent), plusieurs stratégies sont mises en oeuvre. Une fois générée la règle de discrimination ou de régression, l'estimation des erreurs associées à cette règle fournit un outil de validation de celle-ci. La validation croisée permet d'appréhender la robustesse de la méthode de discrimination ou de régression, sans avoir recours à un jeu d'individus à tester, et de clarifier l'incidence d'un nombre restreint de mesures sur le modèle. Chaque jeu de données est aléatoirement subdivisé en plusieurs parties. Les sous-parties sont alors itérativement utilisées soit pour calibrer le modèle, soit pour être soumis à une prédiction du modèle. Pour chaque itération de validation croisée, une partie du jeu de donnée est retirée ; le reste du jeu de données sert à calibrer le modèle. Une fois la règle de décision ou de régression générée avec les échantillons de calibration, les coordonnées des échantillons du jeu test sont alors calculées, ce qui permet d'attribuer une prédiction (dose ou groupe) à ces échantillons tests, et de calculer l'erreur de prédiction. L'erreur de prédiction peut se présenter sous la forme d'une matrice de confusion permettant de calculer un taux d'erreur (ou de bonne prédiction), ou bien sous la forme du calcul d'un coefficient d'erreur, typiquement, Q2.

Dans un deuxième temps, il est également possible de tester la robustesse du modèle en générant aléatoirement une série de jeux de données pour lesquels la variable à expliquer Y a été permutée. Il n'y a donc plus de relation entre la variable à expliquer Y et les variables explicatives X, ce qui correspond en statistiques à la validation par l'hypothèse nulle. Typiquement, une série de plusieurs centaines de modèles suivant l'hypothèse nulle est générée et leur robustesse est évaluée par validation croisée. Il est alors possible de montrer que plus les modèles sont aléatoires, plus il est difficile de construire un modèle prédictif. On peut alors montrer que le modèle initial est significativement différent de la population de modèles aléatoires suivant l'hypothèse nulle, ce qui valide d'autant plus fortement le modèle initial.

Selon un mode de réalisation particulièrement avantageux, on peut utiliser des courbes appelées « Receiver Operating Characteristic », ou simplement ROC (« caractéristique de fonctionnement d'un récepteur » en terminologie anglo-saxonne). Ces courbes, connues de l'homme du métier, sont utilisées pour évaluer la performance d'un classifieur binaire. Une courbe ROC donne le taux de classifications correctes dans un groupe (dit taux de vrais positifs) en fonction du nombre de classifications incorrectes (taux de faux positifs) pour ce même groupe. La courbe est donc incluse dans un carré de coté 1, et passe nécessairement par (0,0) et (1,1). Pour l'appliquer aux modèles générés par l'invention, on va tester des échantillons positifs ou négatifs, et les classer suivant la justesse ou non de la prédiction.

Dans le cas d'un modèle parfait, la courbe ROC passe par (0,1). Dans le cas d'un modèle qui ne donnerait pas de meilleurs résultats que le tirage au sort la courbe ROC serait la droite y=x. Dans le premier cas l'intégrale entre 0 et 1 de la courbe vaut 1, et dans le second cas ½.

On va donc mesurer l'aire sous la courbe, appelée « Area Under the Curve » (AUC), qui correspond mathématiquement à la probabilité que le modèle ait raison lors d'une prédiction. Cette valeur sera ainsi un très bon indicateur de la validité d'un modèle.

Quand le modèle a prouvé sa validité, une étape d'identification de biomarqueurs suit. Elle permet de mieux traduire la réalité de la réponse métabolique, puisqu'elle permet de retrouver les métabolites associés aux composantes du jeu de données retenues pour le modèle, c'est-à-dire les plus caractéristiques de l'exposition à la substance toxique. Cette étape se fait par exemple par lecture par un professionnel des pics associés sur le spectre RMN.

Avantageusement, on déduit de ces biomarqueurs une signature de toxicité spécifique de ladite substance. Il n'y a pas de format caractéristique de signature de toxicité, ce peut être tout simplement une liste de métabolites, chacun caractérisé par un coefficient de corrélation (sens de la variation de la concentration du métabolite par rapport à une situation témoin). Ce peut être également une signature graphique, par exemple un spectre simplifié, réduit aux pics détectés somme essentiels par l'analyse multivariée. L'invention n'est pas limitée à une signature de toxicité en particulier.

Encore plus avantageusement, ces signatures sont stockées dans une base de données afin de constituer une banque de signature. On peut envisager une banque de marqueurs, Chaque biomarqueur étant référence par la liste des substances dans le cas desquelles il a été impliqué. Une telle banque pourra être très utile pour un autre aspect de l'invention qui sera décrit plus bas.

### Résultats expérimentaux

Quatre expériences ont été réalisées suivant le mode de réalisation décrit précédemment afin de montrer l'efficacité du procédé d'évaluation toxicologique selon l'invention :
1- Comparaison des signatures métaboliques entre organes bioartificiels et boites de Pétri ;
2- Comparaison des signatures métaboliques de différents organes bioartificiels (foie C3A, rein MDCK et co-culture foie-rein) ;
3- Comparaison des réponses métaboliques spécifiques des différents organes bioartificiels (foie C3A, rein MDCK et co-culture foie-rein) à la même substance (NH₃) ;
4- Comparaison des réponses métaboliques spécifiques d'un foie bioartificiel à différentes substances (NH₃, DMSO, APAP).

Les résultats des expériences 1 à 4 sont exposés respectivement dans les tables 1 à 4.

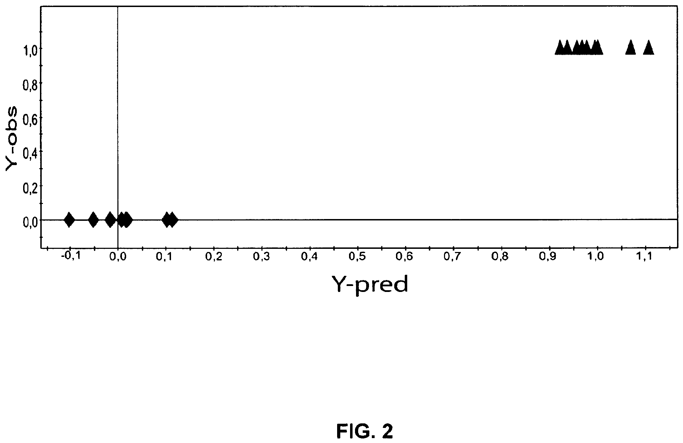

Premièrement, la signature métabolique d'une culture d'organes bioartificiels en bioréacteur a été comparée a celle d'une culture classique en boites de Pétri. Le métabolisme de foies bioartificiels C3A en bioréacteur a été comparé a celui de cellules C3A en culture classique en boite de Pétri. Deux analyses discriminantes aux moindres carrés partiels (PLS-DA) ont été construites afin de comparer la culture de cellules C3A en boites de Pétri a celle de cellules C3A en bioréacteur avec un flux optimal de 10 µL/min (Fig.2). Les modèles PLS-DA montrent une discrimination claire entre les cellules cultivées en boites de Pétri et au sein des bioréacteurs, démontrant une signature métabolique endogène des organes bioartificiels, directement reliée à la culture en bioréacteurs. Les coefficients de chaque modèle PLS ont été analysés afin de repérer les métabolites significativement affectés par la culture en bioréacteur.

La comparaison des listes de métabolites pour chaque condition de culture en bioréacteur (table 1) montre qu'un organe bioartificiel présente une réponse métabolique unique et caractéristique, liée à la physiologie cellulaire d'un organe en trois dimensions, avec une circulation du milieu de culture autour des cellules, ce que ne reproduit pas une culture cellulaire en boîte de Pétri.

La figure 2 illustre également ce résultat en montrant que les boites de Pétri et les bioréacteurs ont différentes signatures métaboliques. Le graphe du nuage des individus montre un écart significatif entre les deux familles.

**Table 1**

| Métabolite | Signe de la corrélation en bioréacteur par rapport a Pétri |
|---|---|
| Lactate | - |
| Formate | - |
| Glucose | + |
| Glycine | + |
| Pyruvate | + |
| Oxaloacetate | + |
| Alpha-hydroxybutyrate | + |

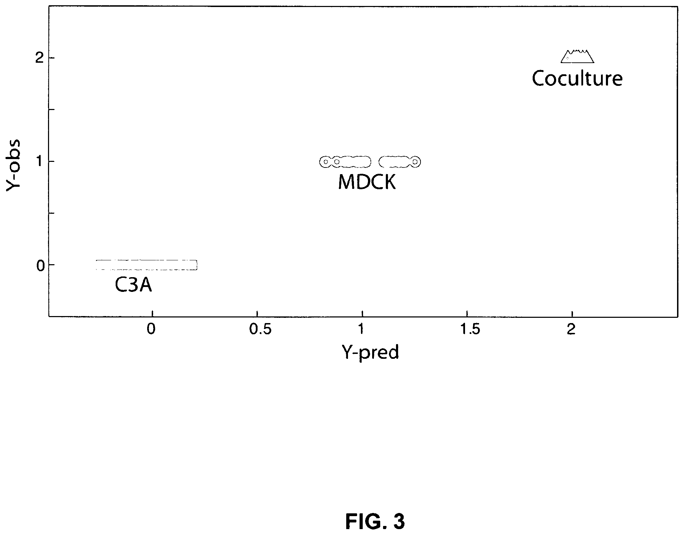

Deuxièmement, les signatures métaboliques des différents organes bioartificiels ont comparées, plus précisément dans le cas du foie (cellules C3A), du rein (cellules MDCK), et de la co-culture foie-rein (C3A/MDCK) en l'absence totale de traitement. Trois analyses discriminantes aux moindres carrés partiels (PLS-DA) ont été construites afin de comparer les cultures de foies bioartificiels C3A aux cultures de reins bioartificiels MDCK et aux co-cultures C3A/MDCK avec un flux optimal de 10 µL/min (Fig.3). Les modèles PLS-DA montrent une ségrégation claire entre les 3 types d'organes. Les coefficients de chaque modèle PLS ont été analysés afin de repérer les métabolites significativement différents entre les organes bioartificiels.

La comparaison des listes de métabolites pour chaque organe bioartificiel révèle une signature métabolique unique démontrant ainsi la présence d'un métabolisme endogène spécifique à chaque type d'organe bioartificiel, directement relié à la fonction physiologique de cet organe dans l'organisme entier (Table 2). Cela est confirmé par la figure 3.

**Table 2**

| Métabolite | Signe de la corrélation C3A | Signe de la corrélation MDCK | Signe de la corrélation C3A-MDCK Co-culture |
|---|---|---|---|
| Glycine | | - | |
| Glutamate | | | + |
| Succinate | + | | + |
| Oxaloacetate | | - | |
| Pyruvate | | - | |
| Pyroglutamate | | + | |
| Alanine | + | | + |
| Valine | + | | |
| Leucine | + | | |
| Isoleucine | + | | |

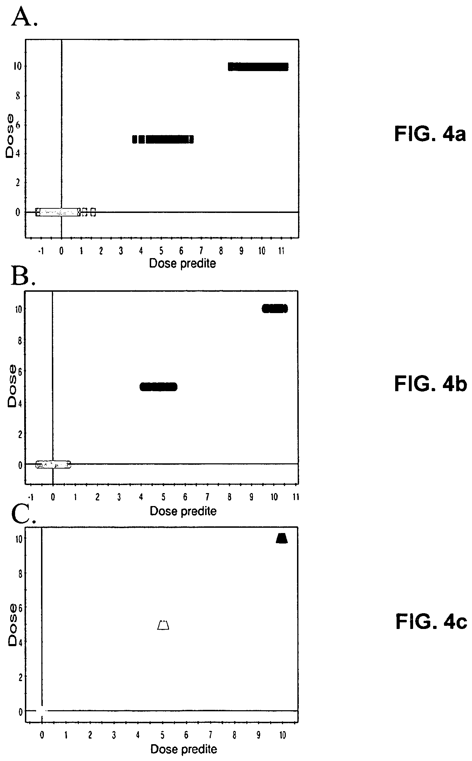

Troisièmement, les réponses métaboliques des différents organes à différentes expositions au NH₃ ont été étudiées. Trois doses de NH₃ ont été administrées (0mM, 5mM et 10mM) à chaque type d'organe bioartificiel. Afin d'augmenter la robustesse du système et s'affranchir des variations métaboliques liées à la densité de culture, celles-ci ont été répliquées plusieurs fois avec différentes densités ; de 200 000 a 1 million de cellules (Figure 4a-c, Table 3). Un modèle de régression multidimensionnelle aux moindres carrés partiels (PLS) a été construit, afin d'identifier une relation quantitative entre la dose NH₃ administrée et la réponse métabolique pour chaque type d'organe bioartificiel. Il en résulte trois modèles PLS spécifiques a chaque type d'organe: foie (C3A, Fig.3A), rein (MDCK, Fig.3B) et co-culture (C3A/MDCK, Fig.3C).

Les modèles de régression PLS montrent une relation quantitative de dose-effet, démontrant une réponse métabolique endogène indirecte quantitativement reliée à la dose de NH3 administrée. Les coefficients de chaque modèle PLS ont été analysés afin de repérer les métabolites significativement affectés par la dose de NH3. La comparaison des listes de métabolites pour chaque organe bioartificiel montre que chacun présente une réponse métabolique unique et caractéristique, liée à la physiologie et toxicologie cellulaire propre à chaque organe.

Dans la table 3, pour chaque métabolite est indiqué le signe de la variation de sa concentration quand la dose de NH3 à laquelle l'organe bioartificiel est exposé augmente.

**Table 3**

| Organe | Métabolites | Variation |
|---|---|---|
| Foie bioartificiel (Cellules C3A) | Alanine | + |
| | Valine | + |
| | Isoleucine | + |
| | Leucine | + |
| | Alpha-keto-methylvalerate | - |
| | Alpha-ketoisovalerate | - |
| | Succinate | + |
| Rein bioartificiel (cellules MDCK) | Glutamine | - |
| | Oxaloacetate | - |
| | Pyruvate | - |
| | Pyroglutamate | + |
| | Lysine | - |
| | Unknown | - |
| Co-culture C3A-MDCK | Glutamine | + |
| | Succinate | + |
| | Glutamate | + |
| | Alpha-hydroxyisobutyrate | + |
| | Valine | + |
| | Lysine | + |
| | ornithine | - |

Quatrièmement, les réponses métaboliques spécifiques de foies bioartificiels exposés à différentes toxines ont été caractérisées (Table 4). Plusieurs substances toxiques (NH3 (0, 5 et 10mM), DMSO (0% 1%, 2% 4%); APAP (0, 1mM)) ont été administrées à des foies bioartificiels constitués de cellules C3A. Dans le cas du traitement par APAP, nous avons comparé la réponse du foie bioartificiel avec celui de cellules de foie en culture dans des boites de Pétri. Pour chaque exposition, un modèle de régression multidimensionnelle aux moindres carrés partiels (PLS) a été construit, afin d'identifier une relation quantitative entre la dose administrée de chaque substance et la réponse métabolique du pour chaque type d'organe bioartificiel. Il en résulte quatre modèles PLS spécifiques a chaque type de traitement: NH₃ sur foie bioartificiel C3A), DMSO sur foie bioartificiel C3A, APAP sur foie bioartificiel C3A et finalement APAP sur culture cellulaire C3A en boites de Pétri. Les modèles de régression PLS montrent une relation quantitative de dose-effet, démontrant une réponse métabolique endogène indirecte des foies bioartificiels quantitativement reliées à chaque traitement administré. Les coefficients de chaque modèle PLS ont été analysés afin de repérer les métabolites endogènes hépatiques significativement affectés par un traitement ou un autre. La comparaison des listes de métabolites pour chaque traitement montre que le foie bioartificiel présente une réponse métabolique unique et caractéristique de chaque molécule toxique administrée (Table 4). Dans cette table, pour chaque métabolite est indiqué entre parenthèses le signe de la variation de sa concentration quand la dose du xénobiotique de l'expérience augmente.

Ces résultats sont chacun une signature de toxicité du xénobiotique, qui lui est propre.

**Table 4**

| NH₃ | DMSO | APAP | APAP pétri |
|---|---|---|---|
| Alanine (+) | | | |
| | Glucose (+) | | |
| Valine (+) | | | |
| Isoleucine (+) | | Isoleucine (-) | Isoleucine (-) |
| Leucine (+) | | | Leucine (-) |
| Succinate (+) | | Succinate (-) | |
| Alpha-keto-methylvalerate (-) | Alpha-keto-methylvalerate (-) | | |
| Alpha-ketoisovalerate (-) | Alpha-ketoisovalerate (-) | | |
| | Phenylalanine (+) | | Phenylalanine (-) |
| | Tyrosine (+) | | |
| | Lactate (-) | | |
| | | Unknown (-) | |
| | | Acetate (+) | |
| | | Tyrosine (+) | |
| | | Pyruvate/oxaloacet ate (-) | |
| | | | Ethanol (-) |
| | | | Glutamate (-) |
| | | | Lysine (-) |
| | | | Arginine (-) |

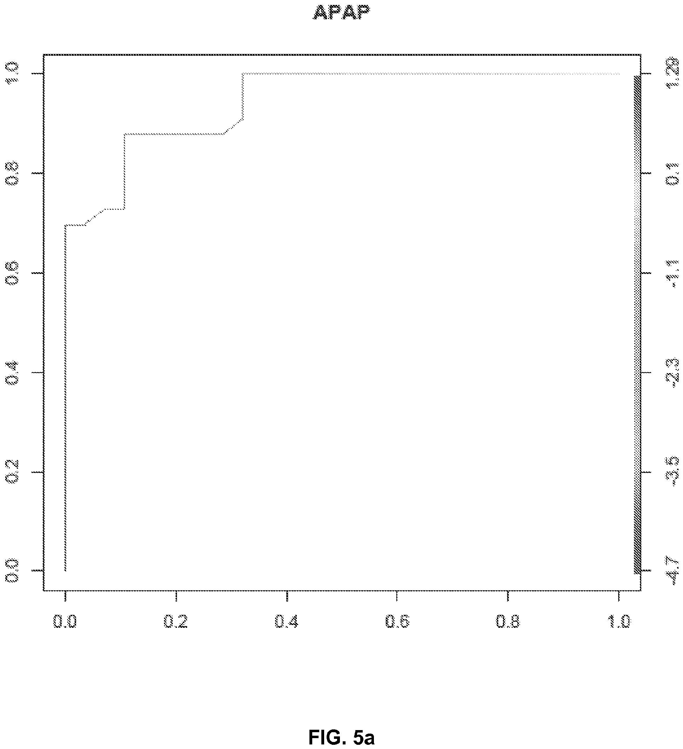

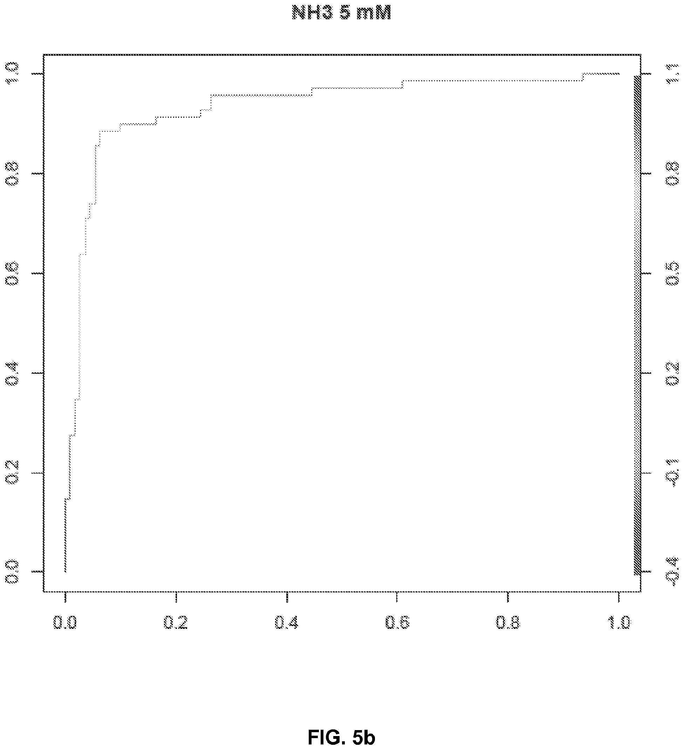

En marge de ces quatre expériences, des courbes ROC ont été calculées pour plusieurs modèles afin d'évaluer leurs performances. Les prédictions de toxicité ont par exemple été faites pour un jeu exposé à de l'APAP à 10mM, et à du NH3 à 5 mM. Les courbes ROC obtenues sont respectivement représentées par les figures 5a et 5b.

L'AUC vaut respectivement 0.943723 et 0.9335968, soit une confiance de près de 95%. Un modèle d'évaluation de toxicité du NH3 à 10mM à même atteint un AUC de 0.9994318 (99.94% de confiance).

Les performances des modèles sont donc pleinement satisfaisantes.

### Criblage toxicologique

L'invention propose selon un deuxième aspect un procédé de criblage toxicologique.

L'idée est de constituer une base de signatures par comparaison avec lesquelles on va pouvoir très rapidement identifier un xénobiotique. Avantageusement, à chaque fois que l'on évalue une nouvelle substance avec le procédé selon le premier aspect, on rajoute sa signature dans une base de données. Au fur et à mesure une base de données de plus en plus complète apparaît.

Lorsque l'on souhaite identifier une substance inconnue, on commence par procéder à l'ensemble des étapes de l'évaluation toxicologique, de façon à obtenir sa signature de toxicité. Ensuite, sont recherchées dans la base de données et identifiées la ou les signatures comparables à la signature de la substance inconnue. Avantageusement, un seuil de tolérance est fixé par l'utilisateur. Un niveau de tolérance très faible augmente la probabilité de ne pas détecter une substance pourtant connue, mais assure un grand degré de confiance si la substance est effectivement identifiée. Un niveau de tolérance plus faible permet de proposer plusieurs solutions, et de laisser l'utilisateur conclure. Alternativement, ce n'est pas les signatures qui sont comparées, mais les listes de biomarqueurs détectés, ou directement les spectres.

Ce procédé permet, si une substance à déjà été rencontrée, de l'identifier avec une grande certitude en quelques minutes seulement, ouvrant des perspectives de criblage haut débit. Actuellement, les seules méthodes d'identification d'une substance inconnue consistent en une succession de tests chimiques décidés par l'utilisateur, nécessitant de faire des hypothèses, et d'y passer du temps et des moyens conséquents.

### Systèmes

L'invention propose selon un dernier aspect des systèmes mettant en oeuvre le procédé d'évaluation toxicologique selon le premier aspect de l'invention, ou mettant en oeuvre le procédé de criblage toxicologique selon le deuxième aspect de l'invention. Ces systèmes comprennent au moins un bioréacteur, des moyens de traitement de données et un dispositif de détection de biomarqueurs.

Pour le système destiné au criblage toxicologique il possède en outre des moyens de stockage de données, sur lequel va pouvoir être stockée la base de données contenant par exemple les signatures des xénobiotiques connus.

## Revendications

1. Procédé d'évaluation toxicologique d'une substance candidate sur au moins un tissu ou organe, **caractérisé en ce qu'**il comprend les étapes de :
(a) Obtention d'un tissu ou organe bioartificiel par simulation ou modélisation de l'activité métabolique dudit tissu ou organe par plusieurs bioréacteurs montés en série ou en parallèle et dans lesquels sont cultivées à la suite différentes familles de cellules qui ensemble simulent le tissu ou l'organe ;
(b) Exposition dudit tissu ou organe bioartificiel à ladite substance ;
(c) Observation de la réponse métabolique du tissu ou de l'organe bioartificiel et acquisition d'un jeu de données multidimensionnel associé sans émettre d'hypothèse concernant la réponse métabolique du tissu ;
(d) Identification au moyen d'une méthode d'analyse statistique multivariée des composantes du jeu de données multidimensionnel quantitativement corrélées à des variables prédéterminées ;
(e) Génération d'un modèle prédictif à partir des composantes du jeu de données effectivement retenues ;
(f) Test du caractère prédictif dudit modèle par au moins une méthode statistique d'estimation de fiabilité ;
(g) Identification de la réponse métabolique du tissu ou de l'organe bioartificiel sous la forme de biomarqueurs associés aux composantes du jeu de données retenues pour le modèle.

2. Procédé selon la revendication précédente, **caractérisé en ce que** la méthode d'analyse multivariée utilisée à l'étape (d) est une analyse discriminante par régression aux moindres carrés partiels (PLS-DA).

3. Procédé selon l'une des revendications précédentes, **caractérisé en ce que** l'étape (d) est précédée d'une étape (d1) de repérage et d'élimination des points aberrants dans ledit jeu de données multidimensionnel.

4. Procédé selon la revendication précédente, **caractérisé en ce que** l'étape (d1) comprend une analyse non supervisée par composantes principales (PCA).

5. Procédé selon l'une des revendications précédentes, **caractérisé en ce que** l'une des méthodes statistiques d'estimation de fiabilité utilisée à l'étape (d) comprend le calcul de l'aire sous une courbe Receiver Operative Characteristic (ROC).

6. Procédé selon l'une des revendications précédentes, **caractérisé en ce que** les variables prédéterminées utilisées à l'étape (d) sont choisies parmi les conditions de culture, le type cellulaire du tissu ou de l'organe bioartificiel, le type de ladite substance, et sa dose.

7. Procédé selon l'une des revendications précédentes, **caractérisé en ce que** l'étape (c) s'effectue par au moins une technique de chimie analytique choisie, parmi les techniques du groupe comprenant la spectroscopie RMN du proton, la spectroscopie RMN du carbone, la spectrométrie de masse, la chromatographie en phase gazeuse, la chromatographie en phase liquide, et les méthodes de détection multiplexées.

8. Procédé selon l'une des revendications précédentes, **caractérisé en ce que** l'étape (c) s'effectue dans le milieu de culture en sortie de bioréacteur afin d'observer la réponse métabolique extracellulaire.

9. Procédé selon l'une des revendications 1 à 7, **caractérisé en ce que** l'étape (c) s'effectue sur les culots cellulaires afin d'observer la réponse métabolique endogène intracellulaire.

10. Procédé selon l'une des revendications précédentes, **caractérisé en ce qu'**il comprend en outre une étape (h) d'obtention d'une signature de toxicité spécifique de ladite substance à partir de la liste des biomarqueurs obtenue à l'étape (g).

11. Procédé selon l'une des revendications précédentes, **caractérisé en ce qu'**il comprend en outre une étape (i) dans laquelle on renseigne une banque de marqueurs et/ou de signatures de toxicité à l'aide des informations obtenues lors des étapes précédentes.

12. Procédé de criblage toxicologique d'une substance candidate sur au moins un tissu ou organe, **caractérisé en ce qu'**il comprend les étapes de :
(a) Obtention d'un tissu ou organe bioartificiel par simulation ou modélisation de l'activité métabolique dudit tissu ou organe par plusieurs bioréacteurs montés en série ou en parallèle et dans lesquels sont cultivées à la suite différentes familles de cellules qui ensemble simulent le tissu ou l'organe ;
(b) Exposition dudit tissu ou organe bioartificiel à ladite substance ;
(c) Observation de la réponse métabolique du tissu ou de l'organe bioartificiel et acquisition d'un jeu de données multidimensionnel associé sans émettre d'hypothèse concernant la réponse métabolique du tissu;
(d) Identification au moyen d'une méthode d'analyse statistique multivariée des composantes du jeu de données multidimensionnel quantitativement corrélées à des variables prédéterminées ;
(e) Génération d'un modèle prédictif à partir des composantes du jeu de données effectivement retenues ;
(f) Test du caractère prédictif dudit modèle par au moins une méthode statistique d'estimation de fiabilité ;
(g) Identification de la réponse métabolique du tissu ou de l'organe bioartificiel sous la forme de biomarqueurs associés aux composantes du jeu de données retenues pour le modèle ;
(h) Obtention d'une signature de toxicité spécifique de ladite substance à partir de la liste des biomarqueurs obtenue à l'étape (g)
(i) Comparaison des biomarqueurs et/ou de la signature de toxicité spécifique avec une banque de marqueurs et/ou signatures de toxicité, de manière à identifier ladite substance.

13. Procédé selon la revendication précédente, **caractérisé en ce que** ladite banque de marqueurs et/ou signatures de toxicité a été établie par la mise en oeuvre d'au moins un procédé selon la revendication 11.

14. Système comprenant une pluralité de bioréacteurs, montés en série ou en parallèle et dans lesquels sont cultivées à la suite différentes familles de cellules qui ensemble simulent le tissu ou l'organe, des moyens de traitement de données et un dispositif de détection de biomarqueurs, **caractérisé en ce qu'**il est adapté pour mettre en oeuvre un procédé d'évaluation toxicologique selon l'une des revendications 1 à 11.

15. Système selon la revendication précédente comprenant en outre des moyens de stockage de données, **caractérisé en ce qu'**il est adapté pour mettre en oeuvre un procédé de criblage toxicologique selon l'une des revendications 12 et 13.

## Patentansprüche

1. Verfahren zur toxikologischen Bewertung einer geeigneten Substanz auf zumindest einem Gewebe oder Organ, **dadurch gekennzeichnet, dass** es die folgenden Schritte umfasst:
(a) Erzielen eines bioartifiziellen Gewebes oder Organs durch Simulation oder Modellierung der Stoffwechselaktivität des Gewebes oder Organs durch mehrere in Reihe oder parallel geschaltete Bioreaktoren, und in denen nacheinander verschiedene Zellfamilien kultiviert werden, die zusammen das Gewebe oder Organ simulieren;
(b) Exposition des bioartifiziellen Gewebes oder Organs gegenüber der Substanz;
(c) Beobachtung der Stoffwechselreaktion des bioartifiziellen Gewebes oder Organs und Erfassung eines zugehörigen multidimensionalen Datensatzes ohne Annahmen bezüglich der Stoffwechselreaktion des Gewebes ;
(d) Identifizierung von Komponenten des multidimensionalen Datensatzes, die quantitativ mit vorbestimmten Variablen korreliert sind, vermittels eines multivariaten statistischen Analyseverfahrens;
(e) Erzeugung eines Vorhersagemodells aus den Komponenten des tatsächlich ausgewählten Datensatzes;
(f) Test des Vorhersagecharakters des Modells durch zumindest ein statistisches Verfahren zur Zuverlässigkeitsschätzung;
(g) Identifizierung der Stoffwechselreaktion des bioartifiziellen Gewebes oder Organs in Form von Biomarkern, die mit den Komponenten des für das Modell ausgewählten Datensatzes verbunden sind.

2. Verfahren nach vorstehendem Anspruch, **dadurch gekennzeichnet, dass** das in Schritt (d) verwendete multivariate Analyseverfahren eine Diskriminanzanalyse durch Regression zu den teilweise kleinsten Quadraten (PLS-DA) ist.

3. Verfahren nach einem der vorstehenden Ansprüche, **dadurch gekennzeichnet, dass** dem Schritt (d) ein Schritt (d1) mit Identifizieren und Eliminieren von Ausreißern im multidimensionalen Datensatz vorausgeht.

4. Verfahren nach vorstehendem Anspruch, **dadurch gekennzeichnet, dass** Schritt (d1) eine nicht überwachte Hauptkomponentenanalyse (PCA) umfasst.

5. Verfahren nach einem der vorstehenden Ansprüche, **dadurch gekennzeichnet, dass** eines der in Schritt (d) verwendeten statistischen Verfahren zur Zuverlässigkeitsschätzung das Berechnen der Fläche gemäß einer Receiver Operative Characteristic (ROC)-Kurve umfasst.

6. Verfahren nach einem der vorstehenden Ansprüche, **dadurch gekennzeichnet, dass** die in Schritt (d) verwendeten vorbestimmten Variablen unter den Kulturbedingungen, dem Zelltyp des bioartifiziellen Gewebes oder Organs, dem Typ der Substanz und ihrer Dosis ausgewählt sind.

7. Verfahren nach einem der vorstehenden Ansprüche, **dadurch gekennzeichnet, dass** Schritt (c) mit zumindest einer Technik der analytischen Chemie durchgeführt wird, die aus der Gruppe bestehend aus Protonen-NMR-Spektroskopie, Kohlenstoff-NMR-Spektroskopie, Massenspektrometrie, Gaschromatographie, Flüssigkeitschromatographie und multiplexierten Erkennungsverfahren ausgewählt ist.

8. Verfahren nach einem der vorstehenden Ansprüche, **dadurch gekennzeichnet, dass** Schritt (c) im Kulturmedium am Ausgang des Bioreaktors durchgeführt wird, um die extrazelluläre Stoffwechselreaktion zu beobachten.

9. Verfahren nach einem der Ansprüche 1 bis 7, **dadurch gekennzeichnet, dass** Schritt (c) an den Zellpellets durchgeführt wird, um die intrazelluläre endogene Stoffwechselreaktion zu beobachten.

10. Verfahren nach einem der vorstehenden Ansprüche, **dadurch gekennzeichnet, dass** es ferner einen Schritt (h) zum Erzielen einer spezifischen Toxizitätssignatur der Substanz aus der Liste der in Schritt (g) erzielten Biomarker umfasst.

11. Verfahren nach einem der vorstehenden Ansprüche, **dadurch gekennzeichnet, dass** es ferner einen Schritt (i) umfasst, in dem eine Bibliothek von Toxizitätsmarkern und/oder -signaturen unter Verwendung der in den vorhergehenden Schritten erzielten Informationen ausgefüllt wird.

12. Verfahren zum toxikologischen Screening einer geeigneten Substanz auf zumindest einem Gewebe oder Organ, **dadurch gekennzeichnet, dass** es die folgenden Schritte umfasst:
(a) Erzielen eines bioartifiziellen Gewebes oder Organs durch Simulation oder Modellierung der Stoffwechselaktivität des Gewebes oder Organs durch mehrere in Reihe oder parallel geschaltete Bioreaktoren, und in denen nacheinander verschiedene Zellfamilien kultiviert werden, die zusammen das Gewebe oder Organ simulieren;
(b) Exposition des bioartifiziellen Gewebes oder Organs gegenüber der Substanz;
(c) Beobachtung der Stoffwechselreaktion des bioartifiziellen Gewebes oder Organs und Erfassung eines zugehörigen multidimensionalen Datensatzes ohne Annahmen bezüglich der Stoffwechselreaktion des Gewebes ;
(d) Identifizierung von Komponenten des multidimensionalen Datensatzes, die quantitativ mit vorbestimmten Variablen korreliert sind, vermittels eines multivariaten statistischen Analyseverfahrens;
(e) Erzeugung eines Vorhersagemodells aus den Komponenten des tatsächlich ausgewählten Datensatzes;
(f) Test des Vorhersagecharakters des Modells durch zumindest ein statistisches Verfahren zur Zuverlässigkeitsschätzung;
(g) Identifizierung der Stoffwechselreaktion des bioartifiziellen Gewebes oder Organs in Form von Biomarkern, die mit den Komponenten des für das Modell ausgewählten Datensatzes verbunden sind;
(h) Erzielen einer spezifischen Toxizitätssignatur der Substanz aus der Liste der in Schritt (g) erzielten Biomarker;
(i) Vergleich von Biomarkern und/oder der spezifischen Toxizitätssignatur mit einer Bibliothek von Toxizitätsmarkern und/oder -signaturen, um die Substanz zu identifizieren.

13. Verfahren nach vorstehendem Anspruch, **dadurch gekennzeichnet, dass** die Bibliothek von Toxizitätsmarkern und/oder -signaturen durch die Durchführung zumindest eines Verfahrens nach Anspruch 11 hergestellt wurde.

14. System umfassend eine Vielzahl von in Reihe oder parallel geschaltete Bioreaktoren und in denen nacheinander verschiedene Zellfamilien kultiviert werden, die zusammen das Gewebe oder Organ simulieren, Datenverarbeitungsmittel und eine Biomarker-Erkennungsvorrichtung, **dadurch gekennzeichnet, dass** es geeignet ist, ein toxikologisches Bewertungsverfahren nach einem der Ansprüche 1 bis 11 durchzuführen.

15. System nach vorstehendem Anspruch, ferner umfassend Datenspeichermittel, **dadurch gekennzeichnet, dass** es geeignet ist, ein toxikologisches Screeningverfahren nach einem der Ansprüche 12 und 13 durchzuführen.

## Claims

1. A method of toxicological evaluation of a candidate substance on at least one tissue or organ, **characterized in that** it comprises the steps of:
(a) obtaining a bioartificial tissue or organ by simulation or modelling of the metabolic activity of said tissue or organ by several bioreactors assembled in series or in parallel and in which are successively cultured various cell families which together simulate the tissue or organ;
(b) exposure of said bioartificial tissue or organ to said substance;
(c) observation of the metabolic response of the bioartificial tissue or organ and acquisition of an associated multidimensional data set without making a hypothesis concerning the metabolic response of the tissue;
(d) identification by means of a method of multivariate statistical analysis of the components of the multidimensional data set which are quantitatively correlated with predetermined variables;
(e) generation of a predictive model on the basis of the components of the data set actually selected;
(f) testing of the predictive nature of said model by at least one statistical method of estimating reliability;
(g) identification of the metabolic response of the bioartificial tissue or organ in the form of biomarkers associated with the components of the data set selected for the model.

2. The method according to the preceding claim, **characterized in that** the method of multivariate analysis used in step (d) is a partial least squares discriminant analysis (PLS-DA).

3. The method according to one of the preceding claims, **characterized in that** step (d) is preceded by a step (d1) of locating and eliminating aberrant points in said multidimensional data set.

4. The method according to the preceding claim, **characterized in that** step (d1) comprises an unsupervised principal component analysis (PCA).

5. The method according to one of the preceding claims, **characterized in that** one of the statistical methods for estimating reliability used in step (d) comprises the calculation of the area under a receiver operating characteristic (ROC) curve.

6. The method according to one of the preceding claims, **characterized in that** the predetermined variables used in step (d) are selected from the culture conditions, the cell type of the bioartificial tissue or organ, the type of said substance, and its amount.

7. The method according to one of the preceding claims, **characterized in that** step (c) is carried out by at least one analytical chemistry technique selected from the techniques of the group comprising proton NMR spectroscopy, carbon NMR spectroscopy, mass spectrometry, gas chromatography, liquid chromatography and multiplexed detection methods.

8. The method according to one of the preceding claims, **characterized in that** step (c) is carried out in the culture medium exiting the bioreactor in order to observe the extracellular metabolic response.

9. The method according to one of claims 1 to 7, **characterized in that** step (c) is carried out on cell pellets in order to observe the intracellular endogenous metabolic response.

10. The method according to one of the preceding claims, **characterized in that** it further comprises a step (h) of obtaining a specific toxicity signature of said substance from the list of biomarkers obtained in step (g).

11. The method according to one of the preceding claims, **characterized in that** it further comprises a step (i) in which a bank of markers and/or toxicity signatures is completed using the information obtained during the preceding steps.

12. A method of toxicological screening of a candidate substance on at least one tissue or organ, **characterized in that** it comprises the steps of:
(a) obtaining a bioartificial tissue or organ by simulation or modelling of the metabolic activity of said tissue or organ by several bioreactors assembled in series or in parallel and in which are successively cultured various cell families which together simulate the tissue or organ;
(b) exposure of said bioartificial tissue or organ to said substance;
(c) observation of the metabolic response of the bioartificial tissue or organ and acquisition of an associated multidimensional data set without making a hypothesis concerning the metabolic response of the tissue;
(d) identification by means of a method of multivariate statistical analysis of the components of the multidimensional data set which are quantitatively correlated with predetermined variables;
(e) generation of a predictive model on the basis of the components of the data set actually selected;
(f) testing of the predictive nature of said model by at least one statistical method of estimating reliability;
(g) identification of the metabolic response of the bioartificial tissue or organ in the form of biomarkers associated with the components of the data set selected for the model;
(h) obtaining a specific toxicity signature of said substance from the list of biomarkers obtained in step (g);
(i) comparison of the biomarkers and/or the specific toxicity signature with a bank of markers and/or toxicity signatures, so as to identify said substance.

13. The method according to the preceding claim, **characterized in that** said bank of markers and/or toxicity signatures was established by the implementation of at least one method according to claim 11.

14. A system comprising a plurality of bioreactors, assembled in series or in parallel and in which are successively cultured various cell families which together simulate the tissue or organ, means of data processing and a device for detecting biomarkers, **characterized in that** it is adapted to implement a method of toxicological evaluation according to one of claims 1 to 11.

15. The system according to the preceding claim further comprising means of data storage, **characterized in that** it is adapted to implement a method of toxicological screening according to one of claims 12 and 13.
